# EUROPÄISCHE PATENTSCHRIFT

(11) **EP 3 728 554 B1**
(45) Veröffentlichungstag und Bekanntmachung des Hinweises auf die Patenterteilung: **20.10.2021**
(21) Anmeldenummer: 18842516.9
(22) Anmeldetag: 10.12.2018
(51) Int. Cl.: C12M 3/00, C12M 1/36

(54) **BIOPROZESS-STEUERVORRICHTUNG SOWIE BIOPROZESS-SYSTEM**
BIOPROCESS CONTROL DEVICE AND BIOPROCESS SYSTEM
DISPOSITIF DE COMMANDE DE BIOPROCÉDÉ AINSI QUE SYSTÈME DE BIOPROCÉDÉ

(30) Priorität: 19.12.2017 EP 17208569
(43) Veröffentlichungstag der Anmeldung: 28.10.2020
(73) Patentinhaber: Eppendorf AG, 22339 Hamburg (DE)
(72) Erfinder: RECKER, Wolfgang, 52066 Aachen (DE); ERTEL, Guido, 41539 Dormagen (DE); SCHNEIDER, Falk, 52072 Aachen (DE); HUFEN, Tristan, 4721 Kelmis (BE)
(74) Vertreter: Patent- und Rechtsanwälte Behrmann Wagner PartG mbB
(86) Internationale Anmeldenummer: PCT/EP2018/084113
(87) Internationale Veröffentlichungsnummer: WO 2019/121089

(56) Entgegenhaltungen:
- EP-A1- 3 188 576
- WO-A1-2008/141659
- WO-A1-2017/032847
- WO-A1-2017/109083
- WO-A2-2004/051267
- WO-A2-2010/025302
- DE-A1-102005 036 763
- DE-A1-102008 049 722
- DE-A1-102012 102 918
- US-A1- 2010 305 759
- US-B1- 6 642 018

## Beschreibung

Die vorliegende Erfindung betrifft eine Bioprozess-Steuervorrichtung nach dem Oberbegriff des Hauptanspruchs. Ferner betrifft die Erfindung ein Bioprozesssystem aufweisend eine derartige Steuervorrichtung, und die Erfindung betrifft ein Verfahren zum Konfigurieren und/oder Umrüsten eines solchen Systems.

Eine derartige Vorrichtung ist aus dem Stand der Technik im Zusammenhang mit dem Betrieb von Bioprozessanlagen allgemein bekannt und dient der konkreten Steuerung und Überwachung von biologischen Versuchs- und Entwicklungsprozessen, wie sie etwa in geeignet eingerichteten Bioreaktoren ablaufen.

So bietet etwa die Anmelderin unter der Marke BioFlo eine Reihe von gattungsgemäßen Bioprozess-Steuervorrichtungen an, die, für vorbestimmte biologische Prozesse konfiguriert, einem Nutzer eine bedienungsfreundliche, betriebssichere und technologisch aktuelle Überwachung und Steuerung der jeweils eingerichteten oder einzurichtenden Prozesse erlauben. Dabei geht der Gattungsbegriff der vorliegenden Erfindung mit den typischerweise in Mehrzahl in einem Vorrichtungsgehäuse aufgenommenen Steuerfunktionseinheiten von einer Vielzahl gattungsgemäß zu steuernder bzw. zu überwachender Bioprozesse aus, von der Entwicklung und (typischerweise allerdings auf kleine Versuchmengen beschränkten) Produktion von Medien wie etwa Impfstoffen, einer Prozessentwicklung im Hinblick auf Zellkulturen oder andere mikrobiologische Anwendungen bis hin zum Klonen und Screenen von Zelllinien und Stammzellenzüchtung reicht die Einsatzbreite der gattungsgemäßen, üblicherweise in Entwicklungsprozessen und anderen, einer großvolumigen Produktion vorgelagerten Prozesstechnologie angesiedelten gattungsbildenden Vorrichtungen. Entsprechend variantenreich sind derartige, gattungsbildend angebotene und typischerweise für einen vorgesehenen Einsatzzweck standardisierte bzw. vorkonfigurierte Systeme, und entsprechend breit gefächert ist die Art und Anzahl der für diese verschiedenen Prozesse einzusetzenden Steuerfunktionseinheiten - diese sind, entsprechend den Anforderungen des jeweiligen Bioprozesses bzw. der Prozessentwicklung, als Steuerungs-, Förder-, Mess- und/oder Sensoreinheiten konfiguriert und üblicherweise einem Bioprozessreaktor geeignet mittels üblicher Leitungen, Schläuche, Kabel od.dgl. zugeordnet, wobei dies üblicherweise auch in unmittelbarer Nachbarschaft, etwa auf einer gemeinsamen Tisch- oder Trägereinheit, erfolgen kann.

Allerdings führt gerade der vorliegende Labor- und Forschungs- bzw. Entwicklungskontext im Einsatz der oberbegrifflichen Bioprozess-Steuervorrichtungen dazu, dass selten lang andauernde Bioprozesse betrieben bzw. gesteuert werden, sodass es, üblicherweise im Bereich von einer bis wenigen Wochen bis hin zu wenigen Monaten, regelmäßiger Re-Konfigurationen bzw. einem Austausch der bekannten Bioprozessor-Steuervorrichtung (im Zusammenhang dann mit einer entsprechenden Anpassung der Bioreaktor-Ausstattung) immer dann bedarf, wenn ein Entwicklungs- bzw. Versuchsprogramm geändert wird.

Bei der als bekannt und gattungsbildend vorausgesetzten Technologie wird dann entweder durch eine geschulte Servicekraft des Systemherstellers eine an einen nächsten, zur Entwicklung und Erprobung anstehenden Bioprozess angepasste Konfiguration und Ausrüstung der Steuervorrichtung vorgenommen; alternativ hat eine Versuchs-Laborumgebung eine Mehrzahl alternativ vorkonfigurierter Steuervorrichtungen lagernd, welche dann geeignet angeschlossen und in Betrieb gesetzt werden.

Der Nachteil dieser als aus dem Stand der Technik bekannt vorauszusetzenden Technologien ist offensichtlich: Insbesondere angesichts zunehmend kürzerer (Entwicklungs-)Prozesszyklen und entsprechend einer kürzeren Abfolge notwendiger Austausch- bzw. Umrüstmaßnahmen ist eine damit verbundene häufige Umrüsttätigkeit geschulten herstellerseitigen Servicepersonals ebenso potentiell unwirtschaftlich, wie das Vorhalten einer Mehrzahl von vorkonfigurierten Einheiten (in jeweiligen Vorrichtungsgehäusen), welche dann überwiegend ungenutzt (und in knappem Laborraum) zu lagern sind, bis eine jeweils geeignet vorhandene konfigurierte Steuervorrichtung benötigt wird.

Diese Herausforderungen sind vor dem Hintergrund sensibler Laborumgebungen zu betrachten, welche nicht nur vor Kontamination und anderen für die Bioprozesse schadensträchtigen Umgebungseinflüssen zu schützen sind, sondern zudem - nicht zuletzt aus qualitätssicherungs- und zulassungsrechtlichen Gründen - das sorgfältige und nachvollziehbare Aufzeichnen und Prüfen der durchgeführten Maßnahmen verlangen.

Weiter ist aus der WO 2017/109083A1 ein Instrument, das zum Bearbeiten von Zellen, zum Beispiel zum Kultivieren, Konzentrieren oder Waschen der Zellen, geeignet ist bekannt, welches ein Gehäuse zur Aufnahme von mechanischen Elementen mit mindestens einer Flüssigkeitspumpe; und einen Einweg-Verarbeitungssatz, der zu den mechanischen Elementen innerhalb des Gehäuses komplementär ist und einen Fluidkreislauf mit einem Fluidspeicher und mehreren Fluidpfaden umfasst, die in der Lage sind, einen durch die Pumpe(n) verursachten Fluidstrom zu führen, wobei das Instrument ferner einen Mechanismus zum Bestimmen der Menge oder Änderung der Menge des Fluids in dem Speicher, die sich aus dem Fluidstrom ergibt, beinhaltet, wobei das Instrument ferner eine Steuerung umfasst, die betreibbar ist, um mindestens die Pumpe zu steuern.

Aus der DE 10 2005 036 763 A1 ist ein System aus mehreren Inkubatoren bekannt, die jeweils ein Gehäuse, eine innerhalb des Gehäuses angeordnete Probenkammer zur Aufnahme eines Probenbehältnisses, eine Temperiereinheit und eine die Temperiereinheit steuernde Steuerelektronik umfassen. Dabei sind die Probenkammern der Inkubatoren mittels der Temperiereinheiten beheiz- und/oder kühlbar und weisen die Gehäuse der Inkubatoren jeweils eine verschließbare Zugangsöffnung zum Be- und Entladen der Probenkammer auf, wobei die Gehäuse der Inkubatoren ein vertikales Stapeln der Inkubatoren gestatten. Dabei weisen die Inkubatoren jeweils ein mit ihrer Steuerelektronik zusammenwirkendes Bussystem auf, wobei die Bussysteme der Inkubatoren über entsprechende Anschlußelemente untereinander verbunden sind. Ferner umfaßt das System eine zentrale Steuereinheit, die mit dem Bussystem eines der Inkubatoren des Systems verbunden ist, so daß über die Bussystemarchitektur ein individuelles Ansteuern der Steuerelektronik sämtlicher Inkubatorendes Systems durch die zentrale Steuereinheit möglich ist.

Die WO 2017/032847 A1 wird eine Bioverarbeitungsvorrichtung mit einem Gehäuse, einer im Wesentlichen geschlossenen Bioreaktorkammer im Inneren des Gehäuses und einem weiteren im Wesentlichen geschlossenen Bereich im Inneren des Gehäuses, der elektrische Teile und/oder elektronische Steuerungskomponenten enthält offebart, wobei die Kammer Folgendes beinhaltet: eine Schale zum Tragen eines Bioreaktors, einen Schalenträger mit einem Mechanismus zum Schaukeln der Schale im Gebrauch der Schale mit einer Heizung zum Kontaktieren eines Bioreaktors und Erwärmen derselben, und die Vorrichtung ferner mit einer Sekundärheizung zum Erwärmen von Luft, die die Schale umgibt.
Die WO 2010/025302 A2 handelt von Systemen, Vorrichtungen, Vorrichtungen und Verfahren zur automatisierten Bioverarbeitung. Protokolle und Bioverarbeitungsverfahren, die für die hierfür geeignet sind, sind unter anderem: Immunpräzipitation, Chromatinimmunpräzipitation, rekombinante Proteinisolierung, Nukleinsäuretrennung und -isolierung, Proteinmarkierung, Trennung und Isolierung, Zelltrennung und -isolierung, Lebensmittelsicherheitsanalyse und automatische perlenbasierte Trennung.

Aufgabe der vorliegenden Erfindung ist es daher, eine Bioprozess-Steuervorrichtung nach dem Oberbegriff des Hauptanspruchs im Hinblick auf ihre Eignung für eine Mehrzahl verschiedener Bioprozesse zu verbessern, dabei insbesondere eine Anpassung bzw. Umrüstung bzw. Konfiguration der Steuervorrichtung für aufeinanderfolgende, verschiedene Bioprozessanwendungen einfacher, komfortabler und ökonomischer zu gestalten, ohne dass eine Gefahr von Fehlbedienungen, anderen Prozessbelastungen oder Beeinträchtigungen durch externe Einflüsse besteht. Zusätzlich sind die Voraussetzungen für eine zuverlässige und überprüfbare Konfiguration sowie einen späteren (Versuchs-)Betrieb derartiger Steuervorrichtungen für Bioprozesse zu schaffen, wobei diese Technologie sich insbesondere durch eine - gegenüber einer Großserienproduktion biologischer und biotechnologischer Produkte - kurze Prozess-, Prozessentwicklungs- und Anpasszeit auszeichnen soll.

Diese Aufgaben werden durch die Bioprozess-Steuervorrichtung mit den Merkmalen des Hauptanspruchs gelöst; vorteilhafte Weiterbildungen der Erfindung sind in den Unteransprüchen beschrieben. Zusätzlich Schutz im Rahmen der Erfindung wird beansprucht für ein Bioprozess-Steuersystem, welches die erfindungsgemäße Bioprozess-Steuervorrichtung sowie eine Mehrzahl der im Rahmen dieser Steuervorrichtung vorgesehenen Steuerfunktionseinheiten aufweist, wobei im Rahmen dieses Systems dann eine Eignung für mindestens zwei alternativ zu steuernde Bioprozesse, durch entsprechendes Auswählen und Austauschen der jeweils erforderlichen Steuerfunktionseinheiten, in einem Vorrichtungsgehäuse ermöglicht sein soll. Dabei ist die Erfindung insbesondere für einen Labor-, Forschungs- und Prozessentwicklungskontext geeignet, bei welchen derartige Re-Konfigurationen, gegenüber stationären Produktionsprozessen, häufiger auftreten und entsprechend die nachfolgend zu erläuternden erfindungsgemäßen Vorteile deutlich zutage treten.

So weist zunächst in erfindungsgemäß vorteilhafter Weise das Vorrichtungsgehäuse der Bioprozess-Steuervorrichtung modulartig ausgebildete Schubladenbaugruppen auf, die jeweils zur Aufnahme von Steuerfunktionseinheiten ausgebildet und vorgesehen sind. Diese Schubladenbaugruppen sind so im Zusammenwirken mit stationären Gehäuserahmenmitteln des Vorrichtungsgehäuses ausgebildet, dass die Schubladenbaugruppen in einer Öffnungs- und/oder Entnahmeposition, welche bevorzugt getrennt oder trennbar von den Gehäuserahmenmitteln ist (und etwa auch ein Entfernen vom Gehäuserahmen ermöglicht), einen Konfigurations- und/der Montagezugriff auf die in der betreffenden Schubladenbaugruppe aufgenommene Steuerfunktionseinheit ermöglichen. In diesem Zustand ist es daher möglich, einer - weiter bevorzugt im Rahmen der Erfindung gleich bzw. standardisiert ausgebildeten - Schubladenbaugruppe eine jeweils gewünschte bzw. notwendige Steuerfunktionseinheit zuzuordnen, üblicherweise durch Verschrauben od.dgl. lösbare Festlegungsmaßnahmen an oder in der Schubladenbaugruppe.

Zusätzlich erfindungsgemäß sind dann die Schubladenbaugruppen im Zusammenwirken mit dem Vorrichtungsgehäuse bzw. den Rahmenmitteln so ausgestaltet, dass, typischerweise durch Einschieben od.dgl. Betätigungsmaßnahmen, die betreffende Schubladenbaugruppe in eine Betriebs- und Verschlussposition verbringbar ist, an welcher mittels erfindungsgemäßer Riegelmittel die Schubladenbaugruppe verriegelnd festlegbar ist. Erfindungsgemäß erfolgt in dieser Position sowohl ein elektrisch versorgender, als auch ein elektrisch funktionsidentifizierender Kontakt zwischen der (in der Schubladenbaugruppe aufgenommenen) Steuerfunktionseinheit und elektrischen Energieversorgungsmitteln (einerseits) sowie Systemidentifikationsmitteln (andererseits), welche entweder den Rahmenmitteln stationär, ergänzend oder alternativ einer weiteren Schubladenbaugruppe zugeordnet und dort vorgesehen bzw. montiert sein können. Erfindungsgemäß weiterbildend sind diese Einheiten so ausgestaltet, dass ein Austausch der Schubladenbaugruppen selbst im laufenden Bioprozess-Steuerungsbetrieb der Vorrichtung erfolgen kann, mit damit erreichter weiterer Optimierung der Flexibilität.

Auf diese erfindungsgemäße Weise ist es damit ermöglicht, in hochgradig flexibler und insbesondere auch durch nicht-geschultes Konfigurationspersonal bedienbarer Weise eine Umrüstung gattungsgemäßer Steuervorrichtungen vorzunehmen, ohne dass etwa eine Gefahr von Fehlkonfigurationen oder eines Fehlbetriebs besteht: Durch die erfindungsgemäße Maßnahme, eine jeweils in das Vorrichtungsgehäuse (mittels der ein einfaches Austauschen ermöglichenden) Schubladenbaugruppe im Hinblick auf die jeweilige Funktion (nämlich die inhärente Funktionalität und den Funktionszweck der betreffenden Steuerfunktionseinheit) zu identifizieren, besteht die Möglichkeit, systemtechnisch (und insbesondere durch die erfindungsgemäßen Systemidentifikationsmittel) diese Steuerfunktionseinheit geeignet zu erkennen, in eine System-Gesamtfunktionalität der Bioprozess-Steuervorrichtung einzubinden und eine geeignete Ansteuerung vorzunehmen, ohne dass es etwa gesonderter Kenntnisse oder Einstellungsmaßnahmen durch die das Umrüsten tätigende Bedienperson bedarf - sogar ungeeignete bzw. fehlerträchtige oder defekte Steuerfunktionseinheiten lassen sich durch entsprechende Identifikation und Reaktion mittels der Systemidentifikationsmittel feststellen und, vor dem Entstehen weiterer Betriebs- und Prozessprobleme, etwa durch entsprechende Signalgabe oder eine Deaktivierung dieser Einheiten, berücksichtigen.

Zusätzlich sorgen die erfindungsgemäßen (und weiter bevorzugt mechanisch wirkenden) Riegelmittel nicht nur für ein mechanisch sicheres (und etwa vor einem unbeabsichtigten Herausziehen bzw. Herausfallen) gesichertes Betreiben der in jeweiligen Schubladenbaugruppen gehaltenen Steuerfunktionseinheiten, auch bieten etwa diese Riegelmittel, welche weiter bevorzugt als mechanische Stab- und/oder Bügeleinheiten ausgestaltet sind, die Möglichkeit, eine nicht korrekt an bzw. in der Betriebs- und Verschlussposition sitzende Schubladenbaugruppe samt Steuerfunktionseinheit mechanisch festzustellen und so potentiellen Verbindungs- bzw. Kontaktproblemen vorzubeugen.

Im Ergebnis ermöglicht daher die vorliegende Erfindung in überraschend einfacher Weise das Adressieren der im vorliegenden Bioprozesskontext auftretenden Anpassungsprobleme an wechselnde Versuchs- und Entwicklungsumgebungen, wobei die vorliegende, durch die Erfindung geschaffene Technologie die Flexibilität und die Anpassbarkeit des im Vorrichtungsgehäuse vorgesehenen bzw. vorzusehenden Systems aus Steuerfunktionseinheiten verschiedenster Bestimmung und Funktionalität in signifikanter Weise erhöht.

Es ist beispielhaft vorgesehen, Steuerfunktionseinheiten zur Montage an oder in Schubladenbaugruppen vorzusehen, welche sich durch eine Vielzahl von Einsatzgebieten und Funktionalitäten unterscheiden - neben (insbesondere modulartigen) Fluidtransportmitteln, wie etwa Pumpenvorrichtungen verschiedenster Art für Flüssigkeiten oder Gase, sind insbesondere auch Pumpensteuervorrichtungen in erfindungsgemäßen Schubladenbaugruppen vorsehbar, welche dann in ansonsten üblicher Weise mit bereits am oder im Reaktorgefäß vorhandenen Pumpen od.dgl. Fluidtransportmitteln kommunizieren. Gleichermaßen gilt dies für Ventilvorrichtungen (oder Ventil-Pumpenkombinationen, für Fluidheiz- und/oder Fluidkühlvorrichtungen), sodass beliebige Fluid-Handlingsaufgaben entsprechend der bewährten bisherigen Technologie, nur mit deutlich erhöhter Flexibilität, im Rahmen der vorliegenden Erfindung einricht-, konfigurier- und betreibbar sind. Gleichermaßen können erfindungsgemäße Steuerfunktionseinheiten Funktionalitäten von Gassensorvorrichtungen, Flüssigkeitssensorvorrichtungen, elektrischen und/oder optischen Messvorrichtungen realisieren, ebenso wie als Steuervorrichtungen etwa für Bioreaktor-Rührmittel od.dgl. mechanische Bewegungsaktuatorik dienen, bis hin zu Funktionalitäten des elektrischen Energieeintrags in Bioreaktoren oder verwandte Behälter. Es wird deutlich, dass, insbesondere auch durch die mit der vorliegenden Erfindung vereinfachte Konfigurier- und Anpassbarkeit an verschiedenste Labor- und Prozessabläufe, eine entsprechend große Bandbreite geeignet einzusetzender Funktionalitäten erreicht ist.

Zur erfindungsgemäßen Funktionsidentifikation zwischen Schubladenbaugruppe und Vorrichtungsgehäuse (bzw. stationären GehäuseRahmenmitteln) weisen die Schubladenbaugruppen gemäß der vorliegenden Erfindung Funktionsidentifikations- und Parametermittel auf, welche, bevorzugt ausgestaltet als Elektronikeinheiten und etwa mittels eines Mikrocontrollers realisiert, auf einer an oder in der Schubladenbaugruppe vorgesehenen elektrischen Leiterplatte ausgebildet sind. Diese erfindungsgemäßen Funktionsidentifikations- und Parametermittel stellen dann über eine elektronische Schnittstelle der Schubladenbaugruppe ― etwa über eine elektrische Kontaktleiste, einen Busanschluss od.dgl. ― die jeweils montierte Steuerfunktionseinheit identifizierende Identifikationsdaten sowie den Steuerfunktionseinheiten zugehörige elektrische Parameterdaten zur Verarbeitung durch die (typischerweise gehäuseseitigen) Systemidentifikationsmittel bereit. Mit dieser konkreten Ausgestaltung der Schubladenbaugruppe ist es damit dann insbesondere vorteilhaft und zusätzlich weiterbildend ermöglicht, einen Funktionstyp, eine Serien- und/oder Versionsnummer, eine benötigte Betriebsspannung oder andere für einen Betrieb der jeweils mit der Schubladenbaugruppe bzw. deren Steuerfunktionseinheit verbundene Daten zu erkennen (wobei, etwa bei Realisierung mittels einer Mikrocontrollereinheit, dies üblicherweise automatisiert und programmunterstützt erfolgt). Diese Identifikations- und Kommunikationsprozesse können insbesondere auch während bzw. nach Abschluss der mechanischen Montagearbeiten erfolgen und zusätzlich, etwa in der oben beschriebenen Art, eine Überprüfung auf eine fehlerhafte Bestückung, nicht mehr aktuelle und/oder für einen jeweils gewünschten Prozess nicht mehr brauchbare Funktionalitäten od.dgl. Fehler durchführen. Über weiterbildungsgemäß vorzusehende Signal-, Kommunikations- und/oder Alarmmittel wären dann insbesondere derartige problematische Konfigurationszustände signalisierbar, ebenso wie auch eine ordnungsgemäße und etwa für einen Laborbetrieb bereits taugliche (und auch technische aktuelle) Konfiguration.

Insbesondere das potentielle Problem einer (Parameter-, Betriebssystem- oder Programm-)Aktualisierung jeweiliger (in Schubladenbaugruppen montierter) Steuerfunktionseinheiten lässt sich zusätzlich erfindungsgemäß weiterbildend durch die Kommunikations- und/oder Aktualisierungsmittel umsetzen, welche weiterbildend den erfindungsgemäßen Funktionsidentifikations- bzw. Parametermitteln so zugeordnet sind, dass als Reaktion auf eine elektronische Erfassung der Identifikations- bzw. Parameterdaten eine Änderung dieser Daten (insbesondere in Form von Aktualisierungsdaten) erfolgen kann. Dabei ist es dann zweckmäßig, eine Speicherung dieser Daten schubladenseitig vorzunehmen, und zwar weiter bevorzugt in Form nicht-flüchtiger Speichereinheiten, sodass für nachfolgende, spätere Konfigurationen und Verwendungen ein derartiger Aktualisierungsprozess nicht erneut notwendig ist.

Im Hinblick auf die gerade in einem Labor-, Forschungs- und Entwicklungsumfeld für biologische und biotechnologische Prozesse wichtige Dokumentation, Überprüfbarkeit und Nachverfolgbarkeit der Verfahren und Verfahrensschritte, eingeschlossen etwaiger Konfigurations- und Umrüstschritte, ist es im Rahmen zusätzlich weiterbildender Ausführungsformen vorteilhaft, den Funktionsidentifikations- und Parametermitteln (also für eine jeweilige Schubladenbaugruppe), ergänzend oder alternativ den Systemidentifikationsmitteln (des Vorrichtungsgehäuses bzw. der stationären Gehäuse-Rahmenmittel) elektronische Prozessprotokollmittel so zuzuordnen, dass diese einem Betrieb und/oder einer Konfiguration entsprechende Betriebs- bzw. Konfigurationsdaten, üblicherweise auch über längere Zeiträume, aufzeichnen, speichern und für eine systemexterne Verarbeitung bereitstellen. Dabei ist es weiterbildungsgemäß vorteilhaft, eine derartige protokollartige Erfassung übergreifend für eine jeweilige Gesamt-Konfiguration in einem Vorrichtungsgehäuse vorzunehmen; ergänzend oder zusätzlich kann dies gleichwohl auch Schubladenbaugruppenindividuell erfolgen, nicht zuletzt als vorteilhaft hier ohnehin eine komfortable elektronische Infrastruktur in Form einer Mikrocontrollerbaugruppe vorhanden ist, sodass auch individuelle Betriebshistorien jeweiliger (montierter) Schubladenbaugruppen, auch über mehrere Vorrichtungsgehäuse hinweg, aufzeichen- und verfolgbar sind.

Mechanisch bieten die erfindungsgemäßen Gehäuserahmenmittel des Vorrichtungsgehäuses eine Mehrzahl von schachtartigen Aufnahmen für die Schubladenbaugruppen so an, dass eine jeweilige Schubladenbaugruppe an mehreren alternativen Positionen in jeweiligen schachtartigen Aufnahmen positioniert werden kann, und insbesondere ohne dass besondere Aufnahme- oder Montageorte in Form vorgegebener Aufnahmen notwendig sind. Damit entsteht ein hohes Maß an Flexibilität in der Konfiguration bzw. im flexiblen Austausch der (jeweiligen Steuerfunktionseinheiten tragenden) Schubladenbaugruppen relativ zu den stationären Rahmenmitteln (wobei als "stationär" im Rahmen der vorliegenden Erfindung der Umstand zu verstehen ist, dass, im Gegensatz zu den ein- und ausschiebbaren und prinzipiell auch entnehmbaren Schubladenbaugruppen, eine mechanische Rahmen- und Aufnahmestruktur der Gehäuserahmenmittel, insbesondere in Form der weiterbildungsgemäß vorgesehenen schachtartigen Aufnahmen, ortsunveränderlich bleibt).

Zusätzlich weiterbildend und vorteilhaft sind die Schubladenbaugruppen im Hinblick auf jeweilige frontseitige Außenflächen so ausgestaltet, dass in der erfindungsgemäßen Betriebs- bzw. Verriegelungsposition eine gemeinsame (zumindest abschnittsweise) durchgängige und weiter bevorzugt plane Gehäuseaußenfläche des Vorrichtungsgehäuses entsteht. Nicht nur wird dadurch bereits visuell erkennbar, dass die Mehrzahl der eingesetzten Schubladenbaugruppen sich in einer ordnungsgemäßen Einbau- und Verriegelungsposition befindet (und nicht etwa hervorsteht), auch eignet sich eine dadurch ermöglichte, durchgängige Außenfläche in besonders bevorzugter Weise zur Behandlung mit Reinigungs- und Desinfektionsmitteln, wie es in einem biologischen bzw. biotechnischen Laborkontext notwendig ist, um die hygienischen und Sauberkeits-Bedingungen sicherzustellen. Zusätzlich weiterbildend sind zu diesem Zweck die frontseitigen Außenflächen der Schubladenbaugruppen insbesondere kachel- bzw. mosaikartig so ausgestaltet und benachbart zueinander angeordnet, dass, mit möglichst geringen Spaltmaßen im Übergang, die gewünschte Durchgängigkeit der Gesamtfläche realisiert ist. Ergänzend bzw. zusätzlich unterstützend lassen sich verbleibende Spalten durch Dichtmittel od.dgl. Zubehör auffüllen, sodass insbesondere zu verwendende flüssige Reinigungsmittel nicht in das Vorrichtungsgehäuse eindringen und die Funktionalität beschädigen können.

In mechanisch und funktional besonders günstiger Weise sind die erfindungsgemäßen Riegelmittel weiterbildungsgemäß so ausgestaltet, dass diese als Riegelbaugruppe mechanisch auf einem Widerlager- und/oder Verriegelungsabschnitt einer Schubladenbaugruppe greifen, insbesondere dort aufgeschoben werden können. Entsprechend entsteht ein sperrender Kraftschluss zwischen der Schubladenbaugruppe und den stationären Gehäuserahmenmitteln, wodurch ein unbeabsichtigtes Herausziehen und damit verbundene Kontakt- und Verbindungsprobleme wirksam verhindert werden können.

Zusätzlich weiterbildungsgemäß ist es vorteilhaft, eine solche Riegelbaugruppe stab- bzw. bügelartig auszubilden und etwa seitlich querab (d.h. in einem Winkel von ca. 90° zur Einführungs- bzw. Ausführungsrichtung der Schubladenbaugruppen relativ zu den Gehäuserahmenmitteln) zu führen. So lässt sich auf diese Weise, etwa mit einer entsprechend mehrarmig ausgestalteten Riegelbaugruppe, mit einem Riegelvorgang gleichzeitig eine Mehrzahl von (etwa typischerweise in Schachtaufnahmen übereinander angeordneten) Schubladenbaugruppen erreichen, auch zeigt dann ein nicht vollständiges Fluchten des Widerlager- bzw. Verriegelungsabschnitts mit einem für den Kraftschluss eingerichteten Rahmenabschnitt einen fehlerhaften Einschubzustand einer Schubladenbaugruppe an, welcher dann das Verriegeln verhindert.

Generell ist es zudem im Rahmen der Erfindung vorteilhaft, die Kontaktgabe, insbesondere zwischen Steuerfunktionseinheiten und Gehäuserahmenmitteln bzw. daran vorzusehenden Kontaktpartnern für die den Schubladenbaugruppen zugeordnete Schnittstellen, geeignet federnd auszugestalten, nicht nur mit dem Zweck eines die Kontaktgüte verbessernden Toleranzausgleichs, sondern auch zum Erreichen minimierter und fehlervermindernder Übergangswiderstände jeweiliger elektrischer Kontakte.

Im Ergebnis gestattet die vorliegende Erfindung das Realisieren flexibler, einfach und kurzfristig umrüstbarer Bioprozess-Steuervorrichtungen für eine große Vielzahl möglicher Einsatzgebiete, wobei, gegenüber dem gattungsbildenden Stand der Technik, die Möglichkeiten eines flexiblen Einsatzes und einer Neu- bzw. Umkonfiguration drastisch verbessert sind (bei gleichzeitiger Minimierung von Fehlern, eingeschlossen Fehlkonfigurationen, fehlerhafter Einrichtung oder fehlerhafter Auswahl von Steuerfunktionseinheiten).

Dabei ist es im Rahmen des erfindungsgemäß zusätzlich beanspruchten Systemgedankens vorteilhaft, nicht nur Steuerfunktionseinheiten für mehr als einen Bioprozess vorzuhalten, auch ist es etwa zusätzlich weiterbildungsgemäß möglich, das erfindungsgemäße Vorrichtungsgehäuse durch zusätzlich und extern vom Gehäuse vorzusehende Gehäuserahmenmittel zu ergänzen, damit diese etwa bei besonders komplexen und einer über die Anzahl von Schubladenpositionen (Schachtaufnahmen) des hauptsächlichen Vorrichtungsgehäuses hinausgehenden Anzahl von Steuerfunktionseinheiten zusätzliche flexible Erweiterungsmöglichkeiten anbietet. Zusätzlich weiterbildend und vorteilhaft kann ein derartiges zusätzliches Vorrichtungsgehäuse (welches erfindungsgemäß auch die Bioprozess-Steuervorrichtung gemäß Hauptanspruch weiterbildet) als passive Gehäuseeinheit realisiert sein, nämlich insbesondere dadurch, dass die in den zusätzlichen gehäuseexternen Gehäuserahmenmitteln aufgenommenen oder aufzunehmenden zusätzlichen Schubladenbaugruppen (samt jeweils zugehöriger zusätzlicher Steuerfunktionseinheiten) durch geeignete (etwa Kabel-gebundene) Verkopplung mit dem Vorrichtungsgehäuse dessen elektrische Versorgungsmittel (also ein zentrales Netzteil) sowie die (zentralen) Systemidentifikationsmittel nutzen.

Zusätzlich Schutz im Rahmen der Erfindung wird beansprucht für ein Verfahren zum Konfigurieren und/oder Umrüsten eines Bioprozesssystems, wie es insbesondere durch mindestens einen Bioreaktor im Zusammenwirken mit der erfindungsgemäßen Bioprozess-Steuervorrichtung sowie den darin in den Schubladenbaugruppen aufgenommenen Steuerfunktionseinheiten realisiert ist. Dieses Verfahren sieht insbesondere den Austausch, das Ergänzen bzw. das Nachrüsten mindestens einer der Steuerfunktionseinheiten (bzw. der zugehörigen Schubladenbaugruppe) für einen Wechsel von einem ersten biologischen, pharmazeutischen, chemischen und/oder biotechnischen Prozess zu einem zweiten derartigen Prozess vor, sodass sich die vorliegende Erfindung insbesondere auch in der signifikanten Zeitreduktion und Flexibilität im Rahmen eines Konfigurations- und Umrüstprozesses auszeichnet. Zusätzlich weiterbildend sieht dieses Verfahren vor, dass als Reaktion auf das Montieren einer Mehrzahl von Schubladenbaugruppen im Vorrichtungsgehäuse, ergänzend oder alternativ als Reaktion auf das Ersetzen der mindestens einen Schubladenbaugruppe, jeweils ein automatisches Identifizieren, Ergänzen oder alternativ ein automatisches Parametrisieren, der jeweils damit montierten Steuerfunktionseinheiten, im Hinblick auf eine zentrale (gehäuseseitige) Steuer- bzw. Identifikationseinheit, realisiert ist.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1:: ein schematisches Blockschaltbild der erfindungsgemäßen Bioprozess-Steuervorrichtung mit wesentlichen Funktionskomponenten gemäß einem ersten Ausführungsbeispiel;
- Fig. 2:: eine Perspektivansicht einer mechanisch-konstruktiven Realisierung der Bioprozess-Steuervorrichtung eines Ausführungsbeispiels mit geöffnetem Gehäuse, einer montierten Mehrzahl von in Schubladenbaugruppen aufgenommenen Steuerfunktionseinheiten und exemplarisch losgelöst dargestellten Baugruppen und
- Fig. 3 bi:
- Fig. 5:: verschiedene Detailansichten des Vorrichtungsgehäuses des Ausführungsbeispiels der Fig. 2, insbesondere der (stationären) Gehäuserahmenmittel im Zusammenwirken mit einer Schubladenbaugruppe sowie den hier bügelartig realisierten Riegelmitteln.

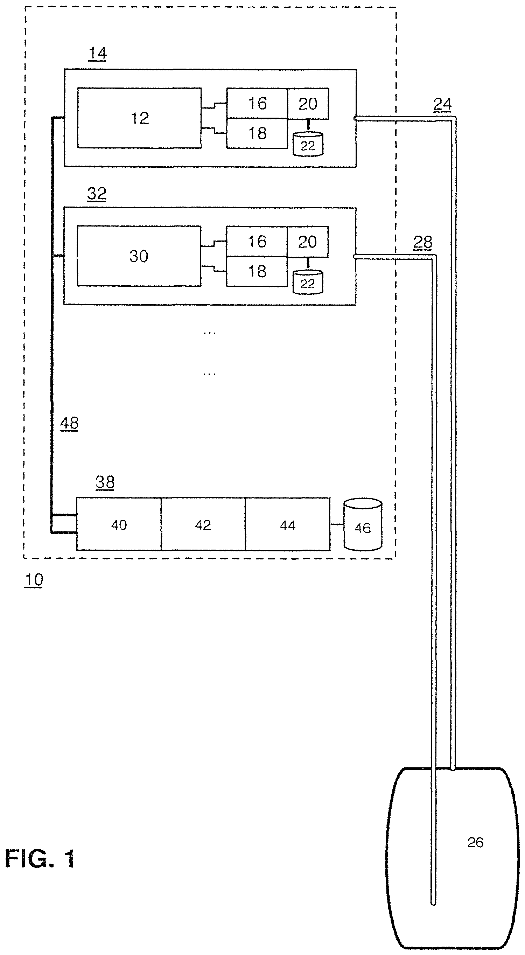

Die Fig. 1 zeigt als Funktions-Blockschaltbild den logisch-funktionalen Aufbau der Bioprozess-Steuervorrichtung gemäß einem ersten Ausführungsbeispiel der vorliegenden Erfindung. Dabei verdeutlicht die gestrichelte Rahmenlinie 10 sowohl ein Vorrichtungsgehäuse, als auch eine darin ausgebildete Rahmenstruktur (Gehäuserahmenmittel), in welchen zunächst Schächte für das lösbare bzw. entnehmbare Aufnehmen von in Schubladenbaugruppen 14, 32 aufgenommenen Steuerfunktionseinheiten 12, 30 ausgebildet sind. Zusätzlich sind an den Gehäuserahmenmitteln plattenartige Gehäusewände od.dgl. weitere Gehäusekomponenten (entfernbar) vorgesehen, ebenso wie eine zentrale Systemelektronik 38, welche in nachfolgend zu beschreibender Weise elektronisch mit den Steuerfunktionseinheiten 12, 30 kommuniziert und diese mit Betriebsspannung versorgt.

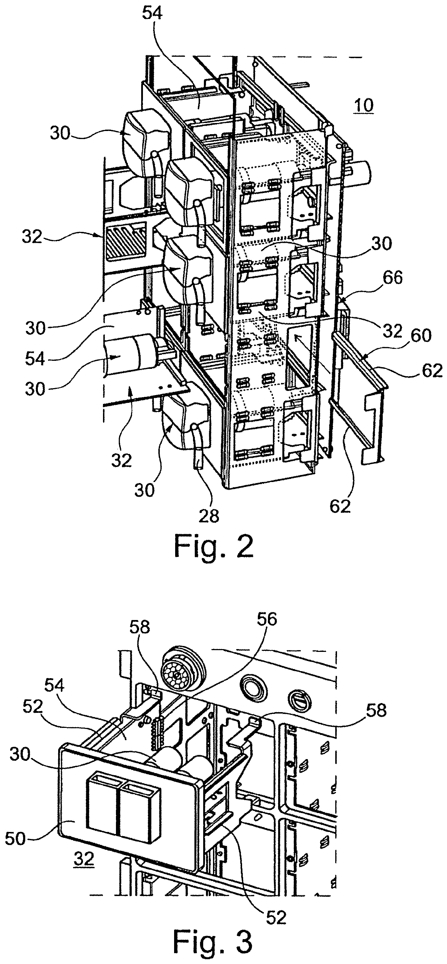

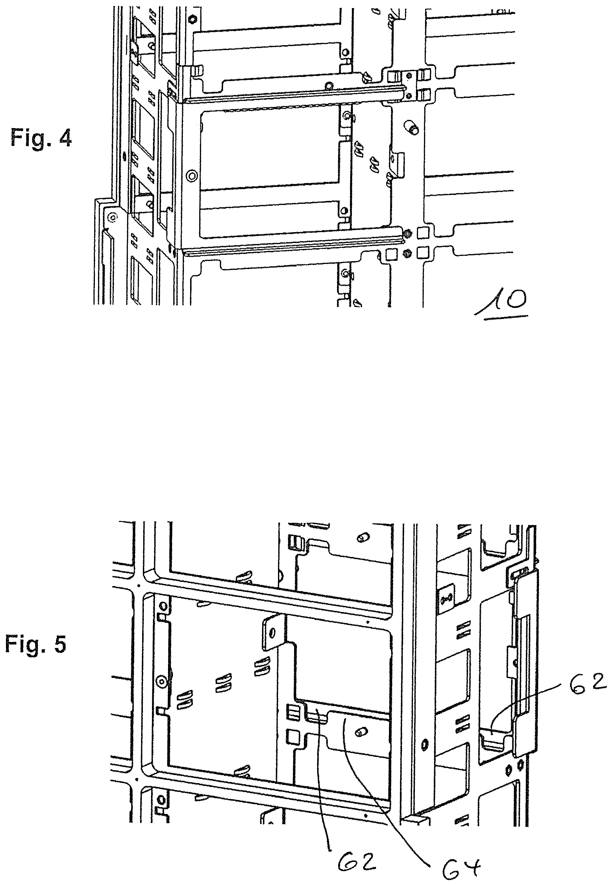

Jede der Schubladenbaugruppen 14, 32 ist mechanisch so gleich bzw. austauschbar ausgebildet, dass diese an beliebigen Einsatz- bzw. Schachtpositionen innerhalb des Gehäuses (vgl. dazu die Beschreibung unten im Zusammenhang mit den Fig. 2 bis 5) eingesetzt werden können. Zu diesem Zweck ist jeder der Schubladenbaugruppen eine (z.B. steckerleistenartige) Schnittstellen-Steckereinheit (56, Fig. 3) zugeordnet, welche in entsprechende, einer jeweiligen Schachtposition des Gehäuses 10 zugeordnete Steckeraufnahmen eingreifen und damit über eine zentrale Versorgungs- und (Daten-)Busleitung an die Versorgungseinheit 38 ankoppelbar ist. In ansonsten üblicher Weise kann zur Realisierung der Verbindung 48 sowohl ein standardisiertes, digitales Bussystem verwendet werden, als auch eine Kommunikation mittels dezidierter und geeigneter Funktionseinheiten zugeordneten Leitungen erfolgen; zusätzlich stellt die Verbindung 48 für jede der Schubladenbaugruppen eine (üblicherweise einheitliche) Versorgungsspannung bereit, welche aus Versorgungsspannungsmitteln 40 der zentralen Versorgungseinheit 38 versorgt wird (welche wiederum in nicht gezeigter Weise mit einem Netzspannungsanschluss verbunden ist).

Jede der einer Schubladenbaugruppe zugeordneten und dort etwa auf einer Trägereinheit der Schubladenbaugruppe montierte Steuerfunktionseinheit ist ausgebildet, eine Funktion im Rahmen des abzubildenden bzw. zu unterstützenden Bioprozesses im Reaktor 26 zu übernehmen bzw. auszufüllen. So ist etwa die Steuerfunktionseinheit 30 als über eine geeignet konfigurierte Fluidleitung 28 mit einem Inneren des Reaktors 26 verbunden, während, wiederum exemplarisch, die Steuerfunktionseinheit 12 als Gas- und Temperatursensor ausgebildet ist, welcher beim schematisch gezeigten Ausführungsbeispiel der Fig. 1 eine Prozessgaskonzentration sowie -Temperatur im Deckelbereich des Reaktors 26 misst.

Im Rahmen des illustrierten Ausführungsbeispiels der vorliegenden Erfindung ist jeder der Steuerfunktionseinheiten zunächst eine Funktionsidentifikationseinheit 16 zugeordnet, welche, als Komponente einer der Schubladenbaugruppe zugeordneten Elektronik (und typischerweise auch mittels einer in jeder Schubladenbaugruppe montierten elektronischen Leiterplatte samt geeignet programmiertem Mikrocontroller sowie Peripherieelektronik realisiert) einerseits den Typ und die konkrete Ausbildung der angeschlossenen Steuerfunktionseinheit, typischerweise nach deren Montage und beim Einsetzen in das Vorrichtungsgehäuse, erkennt und andererseits dann diese Information zur weiteren Berücksichtigung und Verarbeitung an zentrale Systemidentifikationsmittel 42 über das Verbindungssystem 48 zur Verfügung stellt. Damit ist es dann insbesondere einer (nicht näher im Detail gezeigten) zentralen Ablaufsteuerung ermöglicht, zunächst das Vorhandensein der (für den vorliegenden Bioprozess gewünschten oder erforderlichen) Steuerfunktionseinheit festzustellen und dann in einem vorgesehenen Ablauf diese Einheit geeignet zu aktivieren bzw. in der vorgesehenen Weise zu betreiben.

Zusätzlich ist, wiederum als Komponente der Schubladenbaugruppe, jeder Steuerfunktionseinheit eine Parametereinheit 18 zugeordnet, welche, insbesondere im Zusammenwirken mit einer Aktualisierungs- und Konfigurationseinheit 20, das vorbestimmte oder steuerbare Parametrisieren der jeweiligen Steuerfunktionseinheit (12 bzw. 30 im Ausführungsbeispiel der Fig. 1) gestattet: Wiederum bevorzugt durch zentrale Ansteuerung aus der zentralen Systemeinheit 38 ist es damit ermöglicht, sowohl für den Betrieb vorgesehene Betriebsdaten oder andere Parameter zum Konfigurieren oder Betreiben der betreffenden Steuerfunktionseinheit vorzugeben, als auch diese geeignet zu ändern, wenn etwa, durch einen geänderten Prozessablauf Parameteränderungen notwendig sind. Wiederum ergänzend oder alternativ ermöglicht es insbesondere die Baugruppe 20, auch dauerhafte Parameteränderungen, etwa in Form üblicher Systemupdates, durchzuführen, welche dann zusätzlich und vorteilhaft in nicht-flüchtigen Speichermitteln 22, welche gleichermaßen jeder Schubladenbaugruppe zugeordnet sind (und typischerweise auch auf der Schubladen-eigenen Leiterplatte realisiert sind), abgelegt werden können.

Das Ausführungsbeispiel der Fig. 1 verdeutlicht zusätzlich noch eine (hier zentralisierte, nämlich der gemeinsamen, stationären Einheit 38 zugeordnete) Protokolleinheit 44, welche Betriebsdaten (eingeschlossen Konfigurationsdaten und Änderungen dieser), erfasst, in Speichermitteln 46 geeignet ablegt und für Zwecke einer bevorzugt externen Auswertung, etwa einer Qualitätskontrolle od.dgl. Überwachungsmaßnahmen im sensiblen Bioprozessbereich, bereitstellen kann.

Im Gebrauch der in Fig. 1 schematisch dargestellten Vorrichtung ist es dabei (technisch bzw. im Hinblick auf die Hardware-Voraussetzungen nicht gesondert geschulten) Bedienpersonen ermöglicht, bedarfsweise geeignete der Steuerfunktionseinheiten für einen jeweiligen Bio-(Technik-)Prozess auszuwählen, diese Steuerfunktionseinheiten dann in (üblicherweise generischen und austauschbaren) Schubladenbaugruppen zu montieren (alternativ liegen diese vormontierten Schubladenbaugruppen mit entsprechenden Steuerfunktionseinheiten auch schon bereits vor), woraufhin dann die bestückten Schubladenbaugruppen, prinzipiell an beliebigen Positionen, in die Aufnahmeschächte der Rahmenmittel des Vorrichtungsgehäuses eingesetzt werden können. Mit der (nachfolgend zu erläuternden) mechanischen Fixierung erfolgt bevorzugt auch dann eine (weiter bevorzugt federunterstützt realisierte) elektronische bzw. elektrische Kontaktierung, sodass hochgradig flexibel und betriebssicher und mit Minimierung von Umrüstzeiten eine schematisch in der Fig. 1 gezeigte Vorrichtung für eine große Vielzahl von Bioprozess-Anwendungen konfiguriert und umgerüstet werden kann, und wobei sich diese Realisierung, insbesondere angesichts der vorteilhaften Flexibilität und Zeitersparnis beim Umrüsten, besonders bevorzugt für einen Labor-, Entwicklungs- und Forschungskontext eignet (nicht jedoch auf diesen prinzipiell beschränkt ist).

Anhand der Fig. 2 bis 5 wird nachfolgend die exemplarische mechanische Realisierung des schematisch in Fig. 1 gezeigten Ausführungsbeispiels erläutert, wobei hier die in der Perspektivansicht auf das Vorrichtungsgehäuse in Fig. 2 erkennbaren und stirn- bzw. frontseitig hervorstehenden Steuerfunktionseinheiten jeweils Pumpen (30 im generischen Schaltbild der Fig. 1) sind, welche über Fluidleitungen 28 in jeweils vorgesehener Weise in den Reaktorbehälter 26 fluidkommunizierend greifen.

Es wird deutlich, dass jede der Schubladenbaugruppen 32 eine an einer planen Front- bzw. Stirnfläche 50 ansitzende Schubladen-Rahmenstruktur 52 aufweist, welche, in ansonsten bekannter Weise, eine longitudinale Steckbewegung in die in etwa in Fig. 2 erkennbare Schacht- und Rahmenstruktur ermöglicht.

Erkennbar ist insbesondere aus der Perspektivansicht der Fig. 3 die (in der Einschubrichtung) linksseitige Vertikalanordnung einer der Schubladenbaugruppe 32 zugeordneten Leiterplatte 54; deren endseitige Steckeranordnung 56 zeigt ein Beispiel für die Realisierung einer (gefederten) Schnittstellenankopplung zum elektrischen Leitungssystem 48 (Fig. 1).

Zusätzlich erkennbar insbesondere aus der Fig. 3 der teilweise herausgezogenen Schubladenbaugruppe 32 ist ein gehäuseseitiger Winkel- bzw. Vertikalflanschabschnitt 58, welcher im Zusammenwirken mit einer rechteckig-bügelförmigen Riegelbaugruppe (Riegelmittel) 60 hier das erfindungsgemäße Verriegeln einer Schubladenbaugruppe am bzw. im Gehäuse (bzw. den Gehäuserahmenmitteln) bewirkt: Die Riegel- bzw. Verriegelungsbaugruppe 60, bei der in der Fig. 2 gezeigten Art rechtwinklig zur Einschubrichtung der Schubladenbaugruppen und damit von einer Gehäuse- bzw. Rahmenseite des Vorrichtungsgehäuses (bei abgenommener Gehäuseplatte) einzuschieben, greift mit langgestreckten Stababschnitten 62 so in bzw. auf eine Führung 64 der Gehäuserahmenmittel, dass bei eingesetzter Riegelbaugruppe 60 das Paar von Stababschnitten verriegelnd mit den abgewinkelten Flanschabschnitten 58 in Eingriff treten kann.

Erkennbar ist ferner, dass die Mehrzahl der Schubladenbaugruppen im eingeschobenen Zustand mit ihren jeweiligen Frontflächen 50 eine - mit Ausnahme von vorstehenden Abschnitten der jeweiligen Steuerfunktionseinheiten - durchgehende, plane und ununterbrochene Frontfläche des Vorrichtungsgehäuses (Fig. 2) ausbilden, sodass insbesondere damit günstige Voraussetzungen für eine Reinigung, Desinfektion od.dgl. Behandlung im biologischen Laborkontext gegeben ist.

Die in der Figur 1 schematisch als Gruppe mit den Bezugszeichen 38 bezeichnete zentrale Versorgungs-, Identifikations- und Protokollelektronik ist im Ausführungsbeispiel der Figuren 2 bis 5 auf einer der jeweiligen Frontflächen entgegengesetzten rückseitigen Leiterplattenanordnung 66 ausgebildet und ist, wie auch die Seiten- und Ober- bzw. Unterflächen, im Gebrauch durch (geeignet entfernbare) Gehäuseplatten abgedeckt.

Nicht gezeigt ist in den Figuren die Möglichkeit, das Vorrichtungsgehäuse 10 um eine zusätzliche, gehäuseexterne Rahmen- bzw. Gehäusestruktur zu ergänzen, um mögliche weitere und zusätzliche Steuerfunktionseinheiten aufzunehmen, insbesondere für den Anwendungsfall, wenn für einen vorgesehenen Bioprozess die Anzahl der Schächte (und mithin der Aufnahmemöglichkeiten für in Schubladenbaugruppen aufgenommene Steuerfunktionseinheiten) nicht ausreicht und weitere derartige Einheiten insoweit ausgelagert werden müssen. Erfindungsgemäß und vorteilhaft weiterbildend müssen diese zusätzlichen Rahmenmittel jedoch nicht jeweils mit einer eigenen zentralen Elektronik (38 bzw. Leiterplattenausbildung 66) versehen sein; diese kann vielmehr durch eine geeignete weitere Busankopplung, analog der Verbindungsstruktur 48 in Figur 1, erfolgen.

## Patentansprüche

1. Bioprozess-Steuervorrichtung mit
einem Vorrichtungsgehäuse (10) für eine Mehrzahl von aufnehmbaren Steuerfunktionseinheiten (12, 30), die für Bioprozesse ausgebildet und vorgesehen sowie zum Durchführen von Steuerungs-, Förder-, Mess- und/oder Sensorfunktionalitäten konfiguriert oder konfigurierbar und bevorzugt einem Bioprozessreaktor (26) funktional zugeordnet, weiter bevorzugt diesem räumlich benachbart vorgesehen, sein können,
**dadurch gekennzeichnet, dass**
das Vorrichtungsgehäuse zur Aufnahme jeweiliger der Mehrzahl der Steuerfunktionseinheiten modulartig ausgebildete Schubladenbaugruppen (14, 32) aufweist,
die relativ zu stationären Gehäuserahmenmitteln des Vorrichtungsgehäuses in einer Öffnungs- und/oder Entnahmeposition, bevorzugt getrennt oder trennbar von den Gehäuserahmenmitteln, einen Konfigurations- und/oder Montagezugriff auf die in einer betreffenden Schubladenbaugruppe aufnehmbare Steuerfunktionseinheit gestatten,
und in einer durch Riegelmittel (60, 58) verriegelbaren Betriebs- und Verschlussposition einen elektrisch versorgenden sowie elektronisch funktionsidentifizierenden Kontakt zwischen der Steuerfunktionseinheit und den Gehäuserahmenmitteln und/oder einer weiteren Schubladenbaugruppe zugeordneten elektrischen Energieversorgungsmitteln (40) sowie Systemidentifikationsmitteln (42) herstellenwobei mindestens eine der Schubladenbaugruppen Funktionsidentifikations- und Parametermittel (16, 18) aufweist, bevorzugt vorgesehen auf einer elektrischen Leiterplatte (54) der Schubladenbaugruppe,
die an einer elektronischen Schnittstelle der Schubladenbaugruppe eine montierte Steuerfunktionseinheit identifizierende Identifikationsdaten sowie der Steuerfunktionseinheit zugehörige elektrische Parameterdaten zur Verarbeitung durch die Systemidentifikationsmittel (42) bereitstellen.

2. Vorrichtung nach Anspruch 1,
**dadurch gekennzeichnet,**
**dass** die Funktionsidentifikationsmittel (16) ausgebildet sind, einen Funktionstyp, eine Serien- und/oder Versionsnummer, eine Betriebsspannung und/oder eine für den Betrieb der Steuerfunktionseinheit benötigten oder durch diese verarbeitbaren elektrischen Parameter zu erkennen, insbesondere automatisch zu erkennen,
wenn die Funktionseinheit in der Schubladenbaugruppe montiert ist und/oder diese mit montierter Funktionseinheit sich in der Betriebsposition befindet.

3. Vorrichtung nach Anspruch 1 oder 2, **dadurch gekennzeichnet, dass** den Funktionsidentifikations- bzw. Parametermitteln Kommunikations- und/oder Aktualisierungsmittel (20) so zugeordnet sind, dass als Reaktion auf eine elektronische Erfassung der Identifikations- und/oder Parameterdaten eine Änderung dieser Daten, insbesondere in Form von Aktualisierungsdaten, sowie bevorzugt eine Schubladenbaugruppen-seitige Speicherung (22) dieser geänderten Daten erfolgen kann.

4. Vorrichtung nach einem der Ansprüche 1 bis 3,
**dadurch gekennzeichnet,**
**dass** den Funktionsidentifikations- bzw. Parametermitteln (16, 18) und/oder den Systemidentifikationsmitteln (42) elektronische Prozessprotokollmittel (44) so zugeordnet sind, dass diese einem Betrieb und/oder einer Konfiguration insbesondere der Mehrzahl der Steuerfunktionseinheiten entsprechende Betriebsdaten, Konfigurationsdaten und/oder Änderungen dieser Betriebs- und/oder Konfigurationsdaten erfassen, speichern (46) und zur bevorzugt systemexternen Verarbeitung bereitstellen können.

5. Vorrichtung nach einem der Ansprüche 1 bis 4,
**dadurch gekennzeichnet,**
**dass** die Gehäuserahmenmittel des Vorrichtungsgehäuses eine Mehrzahl von schachtartigen Aufnahmen für die Schubladenbaugruppen so anbieten, dass jeweilige der den Schubladenbaugruppen aufgenommenen Funktionssteuereinheiten positionsunabhängig an einer der Aufnahmen platziert und dort in die Betriebs- bzw. Verriegelungsposition verbracht werden können.

6. Vorrichtung nach einem der Ansprüche 1 bis 5,
**dadurch gekennzeichnet,**
**dass** die Mehrzahl der Schubladenbaugruppen in der Betriebs- bzw. Verriegelungsposition mit frontseitigen Außenflächen (50) eine zumindest abschnittsweise durchgängige, bevorzugt plane, Gehäuseaußenfläche des Vorrichtungsgehäuses (10) ausbildet.

7. Vorrichtung nach Anspruch 6,
**dadurch gekennzeichnet,**
**dass** zueinander benachbarte der frontseitigen Außenflächen (50) der Schubladenbaugruppen so in der gemeinsamen Betriebs- bzw. Verriegelungsposition ausgestaltet, kachelartig ausgerichtet und/oder durch zusätzliche Dichtmittel verbindbar sind, dass die durchgängige Gehäuseaußenfläche mit flüssigen Reinigungsmitteln ohne ein Eindringen der Flüssigkeit zwischen die benachbarten Außenflächen behandelbar ist.

8. Vorrichtung nach einem der Ansprüche 1 bis 7,
**dadurch gekennzeichnet,**
**dass** die Riegelmittel als mechanisch auf einem Widerlager- und/oder Verriegelungsabschnitt (58) einer Schubladenbaugruppe (32) greifende Riegelbaugruppe (60) ausgebildet sind,
die bevorzugt bei einer in schachtartige Aufnahmen der Gehäuserahmenmittel eingeschobenen Schubladenbaugruppe einen ein Herausschieben sperrenden Kraftschluss zwischen der Schubladenbaugruppe und den Gehäuserahmenmitteln herstellt.

9. Vorrichtung nach Anspruch 8,
**dadurch gekennzeichnet,**
**dass** die stab- und/oder bügelartig ausgebildete Riegelbaugruppe in einer in einem Winkel größer als 0°, bevorzugt größer 45°, zur Richtung des Einschiebens der Schubladenbaugruppe zur Verriegelung einsetzbar und/oder zum gemeinsamen Verriegeln einer Mehrzahl von eingeschobenen Schubladenbaugruppen ausgebildet ist.

10. Bioprozess-System aufweisend mindestens einen Bioprozessreaktor, die Bioprozess-Steuervorrichtung nach einem der Ansprüche 1 bis 9 sowie eine Mehrzahl der in Schubladenbaugruppen montierbar vorliegenden Steuerfunktionseinheiten, die zum Ausführen von Bioprozessfunktionalitäten am oder im Bioprozessreaktor ausgebildet sind, wobei die Mehrzahl der Steuerfunktionseinheiten so ausgewählt ist, dass mit verschiedenen Teilmengen dieser Mehrzahl mehr als eine Bestückung des Vorrichtungsgehäuses für mehr als einen zu steuernden Bioprozess ermöglicht ist.

11. System nach Anspruch 10,
aufweisend zusätzliche und gehäuseextern vorgesehene oder vorsehbare Gehäuserahmenmittel zur Aufnahme zusätzlicher Schubladenbaugruppen ,
wobei zwischen dem Vorrichtungsgehäuse und den zusätzlichen Gehäuserahmenmitteln elektrische Verbindungsmittel, insbesondere Spannungsversorgungs- und digitale Busmittel, vorgesehen sind, und wobei insbesondere die in den zusätzlichen Gehäuserahmenmitteln aufgenommenen zusätzlichen Schubladenbaugruppen durch die elektrischen Versorgungsmittel sowie die Systemidentifikationsmittel des Vorrichtungsgehäuses versorg- und kontaktierbar ausgebildet sind.

12. Verfahren zum Konfigurieren und/oder Umrüsten eines Bioprozess-Systems nach einem der Ansprüche 10 oder 11, aufweisend die Schritte:
- Montieren einer Mehrzahl der Steuerfunktionseinheiten in zugehörigen Schubladenbaugruppen;
- Montieren einer Mehrzahl von Schubladenbaugruppen im Vorrichtungsgehäuse;
- Betreiben des Bioprozess-Systems und der Bioprozess-Steuervorrichtung zur Durchführung eines ersten biologischen, pharmazeutischen, chemischen und/oder biotechnischen Prozesses in dem mindestens einen Bioprozessreaktor;
- Ersetzen mindestens einer der Mehrzahl von Schubladenbaugruppen durch eine mit einer weiteren Steuerfunktionseinheit versehenen zusätzlichen Schubladenbaugruppe
oder
Hinzufügen der mit der weiteren Steuerfunktionseinheit versehenen zusätzlichen Schubladenbaugruppe zu der Bioprozess-Steuervorrichtung; und
Betreiben des Bioprozess-Systems und der Bioprozess-Steuervorrichtung zur Durchführung eines zweiten, vom ersten Prozess verschiedenen biologischen, pharmazeutischen, chemischen und/oder biotechnischen Prozesses.

13. Verfahren nach Anspruch 12,
**dadurch gekennzeichnet,**
**dass** als Reaktion auf das Montieren mindestens einer der Mehrzahl von Schubladenbaugruppen im Vorrichtungsgehäuse und/oder das Ersetzen oder Hinzufügen Systemidentifikationsmittel die montierte, ersetzte bzw. hinzugefügte Steuerfunktionseinheit mittels der zugehörigen Schubladenbaugruppe identifizieren und/oder mit zugehörigen Betriebsdaten und/oder Betriebsparametern parametrisieren.

## Claims

1. A bioprocess control device having
a device housing (10) for a plurality of receivable control function units (12, 30) designed and provided for bioprocesses and configured or configurable for control, conveying, measuring and/or sensor functionalities and preferably assignable to a bioprocess reactor (26) in a functional manner, more preferably provided spatially adjacent thereto,
**characterized in that**
the device housing has modular drawer assemblies (14, 32) for the reception of respective ones of the plurality of control function units,
the drawer assemblies (14, 32), relative to stationary housing frame means of the device housing in an opening and/or removal position, allowing access for configuration and/or assembly to the control function unit received in a respective drawer assembly, preferably separated or separable from the housing frame means,
and the drawer assemblies establishing an electrically supplying and electronically function-identifying contact between the control function unit and the housing frame means and/or electrical energy supply means (40) and system identification means (42) which are assigned to an additional drawer assembly in an operating or closure position which can be locked by lock means (60, 58), wherein at least one of the drawer assemblies has function identification and parameter means (16, 18), preferably provided on an electrical circuit board (54) of the drawer assembly, which provide identification data which identify an assembled control function unit and electrical parameter data assigned to the control function unit for the processing by the system identification means (42) at an electronic interface of the drawer assembly.

2. The device according to claim 1,
**characterized in that**
the function identification means (16) are realized in order to identify, in particular in an automatic manner, a function type, a serial and/or version number, an operating voltage and/or an electrical parameter which is required for the operation of the control function unit or which can be processed by said control function unit,
if the function unit is assembled in the drawer assembly and/or said drawer assembly and the assembled function unit are in the operating position.

3. The device according to claim 1 or 2, **characterized in that** communication and/or actualization means (20) are assigned to the function identification or parameter means in such a manner that, in response to an electronic recording of the identification and/or parameter data, a modification of said data, in particular in the form of actualization data, and a storage (22) of the modified data, preferably on the side of the drawer assembly, can be realized.

4. The device according to any one of claims 1 to 3,
**characterized in that**
electronic process protocol means (44) are assigned to the function identification or parameter means (16, 18) and/or to the system identification means (42) in such a manner that said process protocol means can record, store (46) and, for the purpose of processing, preferably outside the system, provide operating data, configuration data and/or modifications of said operating and/or configuration data which correspond, in particular, to an operation and/or a configuration of the plurality of control function units.

5. The device according to any one of claims 1 to 4,
**characterized in that**
the housing frame means of the device housing offer a plurality of shaft-like receptions for the drawer assemblies in such a manner that, irrespective of the specific position, respective ones of the function control units received in the drawer assemblies can be placed at one of the receptions and can be displaced into the operating or locking position.

6. The device according to any one of claims 1 to 5,
**characterized in that**
front outer surfaces (50) of the plurality of the drawer assemblies realize an, at least partially continuous, preferably plane, outer surface of the device housing (10) in the operating or locking position.

7. The device according to claim 6,
**characterized in that**
the front outer surfaces (50) of the drawer assemblies which are adjacent to one another can be realized, aligned in a tile-like manner and/or connected by additional sealing means in such a manner in the common operating or locking positon that the continuous outer surface of the housing can be treated with liquid detergents while preventing the liquid from entering between the adjacent outer surfaces.

8. The device according to any one claims 1 to 7,
**characterized in that**
the lock means are realized as a lock assembly (60) which engages on an abutment and/or locking section (58) of a drawer assembly (32) in a mechanical manner, and which preferably realizes a friction-type connection between the drawer assembly and the housing frame means which blocks a pushing out when the drawer assembly is inserted into shaft-like receptions of the housing frame means.

9. The device according to claim 8,
**characterized in that**
the rod- and/or bracket-like lock assembly can be inserted into the drawer assembly for the purpose of locking at an angle of more than 0°, preferably more than 45°, in relation to the direction of the insertion of the drawer assembly and/or that said lock assembly is realized for the common locking of a plurality of inserted drawer assemblies.

10. A bioprocess system comprising at least one bioprocess reactor, the bioprocess control device according to any one of claims 1 to 9 and a plurality of control function units which are provided in the assemblable state in the drawer assemblies, and which are realized in order to perform bioprocess functionalities at or in the bioprocess reactor,
the plurality of control function units being selected in such a manner that different partial quantities of said plurality allow more than one equipping of the device housing for more than one bioprocess to be controlled.

11. The system according to claim 10,
comprising additional housing frame means provided or to be provided outside the housing for receiving additional drawer assemblies,
electrical connection means, in particular voltage supply and digital bus means, being provided between the device housing and the additional housing frame means, and in particular the additional drawer assemblies received in the additional housing frame means being realized so as to be supplied and contacted by the electrical supply means and the system identification means of the device housing.

12. A method for configuring and/or modifying a bioprocess system according to claim 10 or 11, comprising the steps:
- assembling a plurality of control function units in corresponding drawer assemblies;
- assembling a plurality of drawer assemblies in the device housing;
- operating the bioprocess system and the bioprocess control device in order to perform a first biological, pharmaceutical, chemical and/or biotechnical process in the at least one bioprocess reactor;
- replacing at least one of the plurality of drawer assemblies by an additional drawer assembly provided with an additional control function unit
or
adding the additional drawer assembly provided with the additional control function unit to the bioprocess control device; and operating the bioprocess system and the bioprocess control device in order to perform a second biological, pharmaceutical, chemical and/or biotechnical process which is different from the first process.

13. The method according to claim 12,
**characterized in that**,
in response to the assembly of at least one of the plurality of drawer assemblies in the device housing and/or the replacement or addition of system identification means, the assembled, replaced or added control function unit is identified by means of the corresponding drawer assembly and/or is parameterized by corresponding operating data and/or operating parameters.

## Revendications

1. Dispositif de commande de bioprocessus
ayant un boîtier de dispositif (10) pour une pluralité d'unités fonctionnelles de commande (12, 30) recevables, les unités fonctionnelles de commande (12, 30) étant réalisées et prévues pour les bioprocessus et pouvant être configurées ou configurables afin de réaliser des fonctions de commande, de transport, de mesure et/ou de détection et pouvant être de préférence assignées de manière fonctionnelle à un réacteur de bioprocessus (26) et, plus préférentiellement, prévues de manière adjacente audit réacteur de bioprocessus (26),
**caractérisé en ce que**
le boîtier de dispositif a des assemblages de tiroir (14, 32) modulaires pour la réception des unités fonctionnelles de commande respectives de la pluralité d'unités fonctionnelles de commande, les assemblages de tiroir (14, 32) autorisant, par rapport aux moyens de châssis de boîtier fixes du boîtier de dispositif dans une position d'ouverture et/ou de prélèvement, de préférence de manière séparée ou séparable des moyens de châssis de boîtier, un accès de configuration et/ou de montage à l'unité fonctionnelle de commande reçue dans un assemblage de tiroir respectif,
et les assemblages de tiroir, dans une position de fonctionnement et de fermeture verrouillable à l'aide des moyens de verrouillage (60, 58), établissent un contact électrique destiné à identifier la fonction de manière électronique entre l'unité fonctionnelle de commande et les moyens de châssis de boîtier et/ou des moyens d'alimentation en énergie (40) électrique et des moyens d'identification de système (42) assignés à un autre assemblage de tiroir, dans lequel au moins un des assemblages de tiroir a des moyens d'identification de fonction et de paramètre (16, 18), de préférence prévus sur un circuit imprimé (54) électrique de l'assemblage de tiroir, qui fournissent des données d'identification qui identifient une unité fonctionnelle de commande montée et des données de paramètre électriques assignées à l'unité fonctionnelle de commande pour le traitement par les moyens d'identification de système (42) à une interface électronique de l'assemblage de tiroir.

2. Dispositif selon la revendication 1,
**caractérisé en ce que**
les moyens d'identification de fonction (16) sont réalisés afin de détecter, notamment détecter de manière automatique, un type de fonction, un numéro de série et/ou de version, une tension de fonctionnement et/ou un paramètre électrique qui est requis pour le fonctionnement de l'unité fonctionnelle de commande ou qui peut être traité par ladite unité fonctionnelle de commande quand l'unité fonctionnelle est montée dans l'assemblage de tiroir et/ou quand ledit assemblage de tiroir et l'unité fonctionnelle montée sont dans la position de fonctionnement.

3. Dispositif selon la revendication 1 ou la revendication 2, **caractérisé en ce que** des moyens de communication et/ou d'actualisation (20) sont assignés aux moyens d'identification de fonction ou de paramètre de telle manière qu'en réaction à une détection électronique des données d'identification et/ou de paramètre, un changement de ces données, notamment en forme des données d'actualisation, et un stockage (22) des données changées, de préférence du côté de l'assemblage de tiroir, peuvent être réalisés.

4. Dispositif selon l'une quelconque des revendications 1 à 3,
**caractérisé en ce que**
des moyens de protocole de processus (44) électroniques sont assignés aux moyens d'identification de fonction ou de paramètre (16, 18) et/ou aux moyens d'identification de système (42) de telle manière que lesdits moyens peuvent détecter, stocker (46) et, pour le traitement, de préférence en dehors du système, fournir des données de fonctionnement, des données de configuration et/ou des changements de ces données de fonctionnement et/ou de configuration qui correspondent notamment à un fonctionnement et/ou une configuration de la pluralité d'unités fonctionnelles de commande.

5. Dispositif selon l'une quelconque des revendications 1 à 4,
**caractérisé en ce que**
les moyens de châssis de boîtier du boîtier de dispositif offrent une pluralité des logements en forme de puits pour les assemblages de tiroir de telle manière qu'indépendamment de la position, des unités fonctionnelles de commande respectives reçues dans les assemblages de tiroir peuvent être placées à un des logements et peuvent être déplacées dans la position de fonctionnement ou de verrouillage.

6. Dispositif selon l'une quelconque des revendications 1 à 5,
**caractérisé en ce que**
des surfaces externes (50) du côté avant de la pluralité d'assemblages de tiroir réalisent une surface externe du boîtier de dispositif (10), de préférence plate et, au moins par sections, continue dans la position de fonctionnement ou de verrouillage.

7. Dispositif selon la revendication 6,
**caractérisé en ce que**
des surfaces externes (50) adjacentes du côté avant des assemblages de tiroir peuvent être réalisées, alignées en forme de carreau et/ou reliées par des moyens d'étanchéité additionnels dans la position de fonctionnement ou de verrouillage commune de telle manière que la surface externe continue du boîtier peuvent être traitée par des détergents liquides sans que de liquide pénètre entre les surfaces externes adjacentes.

8. Dispositif selon l'une quelconque des revendications 1 à 7,
**caractérisé en ce que**
les moyens de verrouillage sont réalisés comme assemblage de verrouillage (60) qui saisit de manière mécanique sur une partie de butée et/ou de verrouillage (58) d'un assemblage de tiroir (32),
l'assemblage de verrouillage (60) réalisant une liaison par force entre l'assemblage de tiroir et les moyens de châssis de boîtier, de préférence quand un assemblage de tiroir est inséré dans des logements en forme de puits des moyens de châssis de boîtier, la liaison par force bloquant un glissement.

9. Dispositif selon la revendication 8,
**caractérisé en ce que**
l'assemblage de verrouillage réalisé en forme de tige et/ou d'étrier peut être inséré dans l'assemblage de tiroir à un angle de plus de 0°, de préférence plus de 45° par rapport à la direction de l'insertion afin de réaliser le verrouillage et/ou que l'assemblage de verrouillage est réalisé pour le verrouillage commun d'une pluralité d'assemblages de tiroir insérés.

10. Système de bioprocessus ayant au moins un réacteur de bioprocessus, le dispositif de commande de bioprocessus selon l'une quelconque des revendications 1 à 9 et une pluralité d'unités fonctionnelles de commande qui sont prévues à l'état montable dans les assemblages de tiroir et qui sont destinées à réaliser des fonctionnalités de bioprocessus au ou dans le réacteur de bioprocessus,
la pluralité d'unités fonctionnelles de commande étant choisie de telle manière que plus d'un équipement du boîtier de dispositif est permis pour plus d'un bioprocessus à contrôler au moyen des quantités différentes de la pluralité.

11. Système selon la revendication 10,
comprenant des moyens de châssis de boîtier additionnels qui sont ou pouvant être prévus en dehors du boîtier afin de recevoir des assemblages de tiroir additionnels,
des moyens de liaison électrique, notamment des moyens d'alimentation en courant et des moyens de bus digitaux, étant prévus entre le boîtier de dispositif et les moyens de châssis de boîtier additionnels, et notamment les assemblages de tiroir additionnels reçus dans les moyens de châssis de boîtier additionnels pouvant être alimentés et contactés par les moyens d'alimentation électrique et les moyens d'identification de système du boîtier de dispositif.

12. Procédé de configuration et/ou de transformation d'un système de bioprocessus selon la revendication 10 ou la revendication 11, comprenant les étapes :
- monter une pluralité d'unités fonctionnelles de commande dans des assemblages de tiroir correspondants ;
- monter une pluralité d'assemblages de tiroir dans le boîtier de dispositif ;
- fonctionner le système de bioprocessus et le dispositif de commande de bioprocessus afin de réaliser un premier processus biologique, pharmaceutique, chimique et/ou biotechnique dans l'au moins un réacteur de bioprocessus ;
- remplacer au moins un de la pluralité d'assemblages de tiroir par un assemblage de tiroir additionnel pourvu d'une unité fonctionnelle de commande additionnelle
ou
ajouter l'assemblage de tiroir additionnel pourvu de l'unité fonctionnelle de commande additionnelle au dispositif de commande de bioprocessus ; et fonctionner le système de bioprocessus et le dispositif de commande de bioprocessus afin de réaliser un deuxième processus biologique, pharmaceutique, chimique et/ou biotechnique qui se distingue du premier processus.

13. Procédé selon la revendication 12,
**caractérisé en ce**
**qu'**en réaction au montage d'au moins un de la pluralité d'assemblages de tiroir dans le boîtier de dispositif et/ou au remplacement ou l'ajout, des moyens d'identification de système identifient l'unité fonctionnelle de commande montée, remplacée ou ajoutée au moyen d'assemblage de tiroir correspondant et/ou paramètrent ladite unité fonctionnelle de commande en utilisant des données de fonctionnement correspondantes et/ou des paramètres de fonctionnement correspondants.
